Europäisches Patentamt

European Patent Office

Office européen des brevets

(11) Veröffentlichungsnummer: **0 009 215**
**B1**

(12) **EUROPÄISCHE PATENTSCHRIFT**

(45) Veröffentlichungstag der Patentschrift:
06.01.82

(51) Int. Cl.³: **A 61 K  31/66** // (A61K31/66, 31/27)

(21) Anmeldenummer: **79103439.0**

(22) Anmeldetag: **14.09.79**

(54) **Endoparasitizide Pasten für Pferde.**

(30) Priorität: **26.09.78 DE 2841882**

(43) Veröffentlichungstag der Anmeldung:
**02.04.80 Patentblatt 80/7**

(45) Bekanntmachung des Hinweises auf die Patenterteilung:
**06.01.82 Patentblatt 82/1**

(84) Benannte Vertragsstaaten:
**BE CH DE FR GB IT NL SE**

(56) Entgegenhaltungen:
**DE-A-2 423 073**
**US-A-3 937 828**
**US-A-4 141 975**

(73) Patentinhaber: **BAYER AG, Zentralbereich Patente, Marken und Lizenzen, D-5090 Leverkusen 1, Bayerwerk (DE)**

(72) Erfinder: **Dorn, Hubert, Dr., Pahlkestrasse 71, D-5600 Wuppertal 1 (DE)**
Erfinder: **Voege, Herbert, Dr., Martin-Buberstrasse 41, D-5090 Leverkusen 3 (DE)**

## Endoparasitizide Pasten für Pferde

Die vorliegende Erfindung betrifft neue, endoparasitizid wirksame Pasten für Pferde, welche als wirksame Bestandteile ein Gemisch der beiden bekannten Einzelwirkstoffe 2,2,2-Trichlor-1-hydroxyäthyl-phosphorsäuredimethylester ( = Trichlorfon) und N-(2-Methoxyacetamido-4-phenylthiophenyl)-N',N''-bis-methoxycarbonylguanidin ( = Febantel) enthalten.

Trichlorfon ist bekannt (siehe z. B. US-Patent Nr. 2 701 255) und wirkt insbesondere gegen folgende Endoparasiten des Pferdes:

Helminthen wie Askariden, Oxyuren und Habronema spp., außerdem aber auch gegen Gasterophilus spp. (d. h. Insektenlarven, die sich bevorzugt im Maul und im Magen/Darm-Trakt der Pferde aufhalten).

Febantel ist ebenfalls bekannt (siehe z. B. DE-PS Nr. 2 423 679 und US-PS Nr. 3 993 682) und wirkt insbesondere hervorragend gegen Rundwürmer des Magen/Darm-Traktes sowie der Lunge des Pferdes und gegen die Jugendstadien dieser Rundwürmer. Anthelmintische Kombinationen aus Febantel und Trichlorfon sind aus der US-PS Nr. 3 937 825 bekannt.

Durch vorliegende Erfindung werden neue endoparasitizide Pasten für Pferde bereitgestellt, welche als wirksames Prinzip eine Kombination der Einzelwirkstoffe Trichlorfon und Febantel enthalten und deren Wassergehalt unter einem Gewichtsteil bezogen auf die Gesamtmenge des Kombinationspräparats liegt.

Die erfindungsgemäßen endoparasitiziden Pasten für Pferde zeigen überraschenderweise einen synergistischen ( = überadditiven) Effekt gegenüber Endoparasiten und insbesondere gegenüber Strongyliden (Rundwurmart) der Pferde.

Die erfindungsgemäßen Pasten enthalten 5−70 Gewichtsteile Trichlorfon in einer Mindestkristallgröße von 10 µm, 0,5−10 Gewichtsteile Febantel in einer Maximalkorngröße bis zu 30 µm, 20−70 Gewichtsteile flüssige und/oder halbfeste Trägerstoffe, 0−20 Gewichtsteile Zusatzstoffe und weniger als 1 Gewichtsteil Wasser.

Bevorzugte Pasten gemäß vorliegender Erfindung enthalten 20 bis 60 Gewichtsteile Trichlorfon, 2 bis 8 Gewichtsteile Febantel, 30 bis 60 Gewichtsteile flüssige und/oder halbfeste Trägerstoffe, 0 bis 10 Gewichtsteile Zusatzstoffe und weniger als 0,5 Gewichtsteile Wasser.

Als flüssige und/oder halbfeste Trägerstoffe werden bevorzugt Paraffinkohlenwasserstoffe, vorzugsweise Mineralöle, z. B. Paraffinöl eingesetzt.

Unter dem Begriff »Mineralöl« sind alle aliphatischen Kohlenwasserstoffe mineralischen Ursprungs zu verstehen. Mineralöl wird gegenüber pflanzlichen Ölen der Vorzug gegeben, da es nicht ranzig wird, nicht verdaut wird und die Stabilität der Wirkstoffe darin besser ist. Die eingesetzten Mineralöle (z. B. Paraffin) können flüssig oder halbfest sein (z. B. Vaseline) und enthalten bevorzugt aliphatische Kohlenwasserstoffketten mit 16 bis 60 Kohlenstoffatomen.

Als Zusatzstoffe kommen Verdickungsstoffe, Stabilisatoren, Netzmittel und/oder Emulgatoren, Aroma- und/oder geschmacksverbessernde Substanzen in Frage.

Als Verdickungsstoffe seien aufgeführt: anorganische Verdickungsmittel wie Silikate, z. B. Bentonite oder kolloidale Kieselsäure, organische Verdickungsmittel wie Fettalkohole, Fettsäureester (wie z. B. hydriertes Rizinusöl).

Als Stabilisatoren seien Antioxydantien (z. B. Butylhydroxyanisol) aufgeführt.

Als Netzmittel kommen beispielsweise in Frage: Natriumlaurylsulfat.

Als Emulgatoren kommen in Frage, beispielsweise Alkohole wie: Äthylstearylalkohol, Sorbitan-Fettsäureester, Polyoxyäthylen-Sorbitan-Fettsäureester, Polyoxyäthylen-Fettsäureester, Mono- und diglyceride, Lecithin oder Gemische daraus.

Als Aroma- und/oder geschmacksverbessernde Stoffe seien aufgeführt: ätherische Pflanzenöle, z. B. aus Fenchel, Anis.

Zusatzstoffe und insbesondere Verdickungsstoffe werden zur Herstellung der erfindungsgemäßen endoparasitiziden Pasten verwendet, wenn als Trägerstoffe flüssige Trägerstoffe (z. B. flüssige Paraffine) eingesetzt werden. In diesem Fall sind dann diese Verdickungsstoffe zur Erzielung der pastenartigen Konsistenz erforderlich.

Unter Paste soll eine weiche, halbfeste Formulierung verstanden werden, die in einem flüssigen Medium einen unlöslichen Feststoff als disperse Phasen enthält. Die Paste soll im Maul des Pferdes möglichst haften. Die sich im Maul des Pferdes aufhaltenden Gasterophilus-Larven werden durch die Pastenform besonders gut erfaßt, da ein Teil der Paste sich im Maul des Tieres verteilt und die Larven dort abgetötet werden.

Der Wassergehalt der erfindungsgemäßen endoparasitiziden Pasten kann nach an sich bekannten Methoden (z. B. der Karl-Fischer-Titration) bestimmt werden.

Die bei der Herstellung der erfindungsgemäßen endoparasitiziden Pasten eingesetzten Wirkstoffe werden bevorzugt in filgenden Korngrößen verwendet:

Trichlorfon: Mindestgröße der Kristalle: ca. 10 μm, vorzugsweise werden Kristalle mit einer Größe von 30 bis 200 μm eingesetzt.

Febantel: Maximale Korngröße: bis ca. 30 μm, vorzugsweise werden Korngrößen von 0,5 bis 10 μm eingesetzt.

Die Herstellung der endoparasitiziden Pasten erfolgt in an sich bekannter Weise, indem man in Abhängigkeit von der Natur der flüssigen und/oder halbfesten Trägerstoffe sowie der gegebenenfalls verwendeten Zusatzstoffe die Bestandteile in einer beliebigen oder auch bestimmten Reihenfolge miteinander vermischt.

Besonders bevorzugt sind die aus den nachfolgenden Beispielen 1 bis 3 ersichtlichen Herstellungsweisen.

Die erfindungsgemäßen endoparasitiziden Pasten werden jeweils so dosiert, daß durch die Paste (vorzugsweise durch Injektion mit Hilfe eines Pasteninjektors in das Maul des Pferdes) pro kg Körpergewicht des Pferdes 10 bis 50, vorzugsweise 15 bis 40 mg Trichlorfon und 0,1 bis 10, insbesondere 1 bis 6 mg Febantel appliziert werden. In der Regel ist eine Einmaldosierung zur Erzielung des gewünschten Effektes (d. h. Abtötung der sich vor allem im Maul sowie im Magen/Darm-Trakt und in der Lunge des Pferdes aufhaltenden Endoparasiten) ausreichend.

Beispiel 1

Bestandteile einer beispielhaften Pastenpräparation gemäß vorliegender Erfindung:

A) Febantel-Wirkstoff
   (durchschnittliche Teilchengröße: 2 – 5 μm)          8,6 kg
B) Trichlorfon-Wirkstoff
   (durchschnittliche Teilchengröße: 50 – 100 μm)       47,9 kg
C) Emulgierendes Wachs auf Basis
   Äthylstearylalkohol im Gemisch mit Natriumlaurylsulfat
   (Emulsifying Wax BP 73)      ·                        2,5 kg
D) Kolloidale Kieselsäure                                2,6 kg
E) Vaseline (weiß)                                       1,5 kg
F) Dünnflüssiges Paraffin (gemäß DAB VII)*)              46,4 kg
                                                        _____

                                Gesamtmenge              109,5 kg

Diese 109,5 kg entsprechen 100 Liter Pastenpräparation.

*) DAB VII: Deutsches Arzneibuch, 7. Ausgabe, Stuttgart, 1968

Die Pastenformulierung mit den oben angegebenen Bestandteilen wird wie folgt hergestellt:

1) Das emulgierende Wachs (C), die weiße Vaseline (E) und das dünnflüssige Paraffin (F) werden in einen heizbaren mit Rührwerk versehenen Kessel eingetragen.
2) Die Mischung aus C, E und F wird auf ungefähr 80° C erwärmt und so lange gerührt, bis eine homogene Lösung entstanden ist.
3) Anschließend wird unter Rühren auf eine Temperatur von ca. 60° C abgekühlt.
4) Der Wirkstoff Trichlorfon (B) wird zur nach 3) erhaltenen Lösung gegeben und mit einem Rotor-Stator-Dispergierer (z. B. Ultra-Turrax) homogen verteilt.
5) Der Wirkstoff Febantel (A) und die kolloidale Kieselsäure (D) werden zu 4) gegeben und darin homogen verteilt.
6) Die Paste wird über einen Homogenisator gegeben.
7) Anschließend wird die Paste vorzugsweise in Injektoren zur Applikation abgefüllt.

Beispiel 2

Bestandteile:

A) Febantel-Wirkstoff
   (durchschnittliche Teilchengröße: 2 – 5 μm)          8,6 kg
B) Trichlorfon-Wirkstoff
   (durchschnittliche Teilchengröße: 50 – 100 μm)       41,1 kg
C) Emulgierendes Wachs auf Basis Äthylstearylalkohol im

Gemisch mit Natriumlaurylsulfat (Emulsifying Wax BP 73)    2,5 kg
D)   Kolloidale Kieselsäure    2,6 kg
E)   Vaseline (weiß)    1,5 kg
F)   Dünnflüssiges Paraffin (gemäß DAB VII)    52,7 kg
_____

Gesamtmenge    109,0 kg

Die Herstellung und Abfüllung der Paste geschieht in Analogie zu Beispiel 1.

## Beispiel 3

Bestandteile:

A)   Febantel-Wirkstoff
(durchschnittliche Teilchengröße: 2 – 5 µm)    4,3 kg
B)   Trichlorfon-Wirkstoff
(durchschnittliche Teilchengröße: 50 – 100 µm)    20,5 kg
C)   Dickflüssiges Paraffin nach DAB VII    50,0 kg
D)   Vaseline (weiß)    10,0 kg
_____

Gesamtmenge    84,8 kg

Die Vaseline (D) wird im dickflüssigen Paraffin (C) unter Erwärmen auf 30 bis 40°C gelöst. In die entstehende Lösung werden die Bestandteile A (Febantel-Wirkstoff) und B (Trichlorfon-Wirkstoff) langsam eingerührt, anschließend wird im Homogenisator homogenisiert und die Paste im Injektoren zur Applikation abgefüllt.

## Beispiel 4

In einem vergleichenden Versuch werden an stark durch Strongyliden (Rundwurmart) infizierten Pferden unter Verwendung eines Dosiergerätes (sogenannter »Dosierinjektor«) drei Pastenformulierungen vergleichend geprüft. Es handelt sich dabei jeweils um Pastenformulierungen, welche als Wirkstoff

a)   nur Trichlorfon
b)   nur Febantel,
c)   ein Trichlorfon/Febantel-Gemisch gemäß Erfindung

enthalten.
Die Pastengrundlauge ist in den Fällen a), b) und c) die gleiche, sie besteht aus den Komponenten C), D), E) und F) gemäß Beispiel 2. Das Wirkstoffverhältnis in der Pastenformulierung c) beträgt 1 Gewichtsteil Febantel und 30 Gewichtsteile Trichlorfon.
Von den Pasten a), b) und c) werden im Pferdeversuch mit Hilfe des Dosisinjektors gerade so viel appliziert, daß von Trichlorfon 30 mg Wirkstoff pro kg Körpergewicht des Pferdes und von Febantel 1 mg Wirkstoff pro kg Körpergewicht des Pferdes eingesetzt werden.
Der Wirksamkeitsnachweis wird mit Hilfe des Strongyliden-Eireduktionstestes ermittelt. Dabei wird die Wirksamkeit gegenüber großen und kleinen Strongyliden des Pferdes erfaßt und ein beachtlicher synergistischer (d. h. überadditiver) Effekt auf die Reduktion der Parasiten bei Anwendung der erfindungsgemäßen Trichlorfon/Febantel-Paste c) im Vergleich zu den Monopasten a) und b) erzielt.

## Patentansprüche

1. Endoparasitizide Pasten für Pferde, enthaltend 2,2,2-Trichlor-1-hydroxyäthyl-phosphorsäuredimethylester (Trichlorfon) und N-(2-Methoxyacetamido-4-phenylthiophenyl)-N',N''-methoxycarbonylguanidin (Febantel), dadurch gekennzeichnet, daß sie
5 bis 70 Gewichtsteile Trichlorfon in einer Mindestkristallgröße von 10 µm,
0,5 bis 10 Gewichtsteile Febantel in einer Maximalkorngröße bis zu 30 µm,
20 bis 70 Gewichtsteile flüssige und/oder halbfeste Trägerstoffe,
0 bis 20 Gewichtsteile Zusatzstoffe und weniger als
1 Gewichtsteil Wasser enthalten.
2. Endoparasitizide Pasten gemäß Anspruch 1, dadurch gekennzeichnet, daß sie
20 bis 60 Gewichtsteile Trichlorfon,

2 bis 8 Gewichtsteile Febantel,
30 bis 60 Gewichtsteile flüssige und/oder halbfeste Trägerstoffe,
0 bis 10 Gewichtsteile Zusatzstoffe und weniger als
0,5 Gewichtsteile Wasser enthalten.

## Claims

1. Endoparasiticidal pastes for horses containing 2,2,2-trichloro-1-hydroxyethyl-phosphoric acid dimethyl ester (Trichlorfon) and N-(2-methoxyacetamido-4-phenylthiophenyl)-N',N''-methoxycarbonylguanidine (Febantel), characterised in that they contain 5 to 70 parts by weight of Trichlorfon in a minumum crystal size of 10 μm, 0.5 to 10 parts by weight of Febantel in a maximum particle size of up to 30 μm, 20 to 70 parts by weight of liquid and/or semi-solid carriers, 0 to 20 parts by weight of additives and less than 1 part by weight of water.

2. Endoparasiticidal pastes according to Claim 1, characterised in that they contain 20 to 60 parts by weight of Trichlorfon, 2 to 8 parts by weight of Febantel, 30 to 60 parts by weight of liquid and/or semi-solid carriers, 0 to 10 parts by weight of additives and less than 0.5 part by weight of water.

## Revendications

1. Pâtes endoparasiticides pour chevaux, contenant de l'ester diméthylique d'acide 2,2,2-trichloro-1-hydroxyéthyl-phosphorique (Trichlorfon) et de la N-(2-méthoxyacétamido-4-phénylthiophényl)-N',N''-méthoxycarbonylguanidine (Febantel), caractérisées en ce qu'elles contiennent 5 à 70 parties en poids de Trichlorfon en une grandeur de cristal minimale de 10 μm, 0,5 à 10 parties en poids de Febantel en une grandeur de grain maximale allant jusqu'à 30 μm, 20 à 70 parties en poids de matières de support liquides et/ou semi-solides, 0 à 20 parties en poids d'additifs et moins de 1 partie en poids d'eau.

2. Pâtes endoparasiticides selon la revendication 1, caractérisées en ce qu'elles contiennent
20 à 60 parties en poids de Trichlorfon,
2 à 8 parties en poids de Febantel,
30 à 60 parties en poids de matières de support liquides et/ou semi-solides,
0 à 10 parties en poids d'additifs et moins de
0,5 partie en poids d'eau.